Europäisches Patentamt

European Patent Office

Office européen des brevets

(11) Numéro de publication: **0 283 343**
**A1**

(12) **DEMANDE DE BREVET EUROPEEN**

(21) Numéro de dépôt: 88400375.7

(22) Date de dépôt: 19.02.88

(51) Int. Cl.⁴: **B 01 J 29/38**
C 07 C 5/27, C 10 G 45/64

(30) Priorité: 06.03.87 FR 8703223

(43) Date de publication de la demande:
21.09.88 Bulletin 88/38

(84) Etats contractants désignés:
AT BE DE FR GB IT NL

(71) Demandeur: INSTITUT FRANCAIS DU PETROLE
4, Avenue de Bois-Préau
F-92502 Rueil-Malmaison (FR)

(72) Inventeur: Travers, Christine
12, Bd du Général De Gaulle
F-92500 Rueil Malmaison (FR)

Bournonville, Jean-Paul
43, rue des Groues Vauréal
F-95000 Cergy Pontoise (FR)

Franck, Jean-Pierre
24, avenue Ivan Tourgueneff
F-78300 Bougival (FR)

(54) Procédé d'activation d'un catalyseur d'isomérisation de paraffines normales.

(57) Procédé de régénération ou d'activation d'un catalyseur d'isomérisation de n-paraffines, renfermant au moins un métal du groupe VIII supporté par une mordénite acide, dans lequel ledit catalyseur ayant au moins en partie perdu son activité initiale, est traité dans une première étape par un gaz contenant de l'oxygène à une température inférieure à environ 550°C de manière à éliminer la majeure partie du coke contenu sur ledit catalyseur, et dans une deuxième étape le produit issu de la première étape est oxychloré à une température d'environ 200 à 500°C à l'aide d'un mélange gazeux contenant de l'oxygène, de l'eau, et du chlore ou au moins un composé chloré, la quantité de chlore ou de composé chloré employée représentant au total 0,5 à 10 % en poids calculé en poids de chlore par rapport au poids de mordénite.

EP 0 283 343 A1

## Description

La présente invention concerne un procédé de régénération de catalyseurs d'isomérisation, modérément ou sévèrement désactivés, comprenant au moins un métal du groupe VIII supporté par une mordénite sous forme acide.

L'isomérisation des paraffines normales de faible poids moléculaire, est d'une importance considérable dans l'industrie pétrolière vu l'indice d'octane particulièrement élevé des isoparaffines formées.

La modification des législations des principaux pays industrialisés, en ce qui concerne les normes de qualité des essences automobiles et la suppression progressive de l'autorisation d'utiliser des additifs à base de plomb, conduit les producteurs de ces produits à rechercher des procédés améliorés permettant d'obtenir des essences automobiles, sans plomb, à haut indice d'octane.

Les procédés permettant de transformer les paraffines normales, ayant par exemple 4, 5, 6 ou 7 atomes de carbone par molécule, et en particulier les n-paraffines à 5 et 6 atomes de carbone par molécule, en un produit contenant une proportion élevée d'isoparaffines sont particulièrement intéressants.

Ces procédés permettent, en particulier, d'améliorer l'indice d'octane des fractions d'essence légère telles que celles issues, par exemple, du réformage catalytique ou de la distillation directe.

Le mécanisme de la réaction d'hydroisomérisation est habituellement considéré comme un mécanisme bifonctionnel pour lequel il est préférable d'utiliser des catalyseurs comportant à la fois des sites acides et des sites ayant une fonction hydrogénante/déshydrogénante.

Depuis une vingtaine d'années de très nombreuses publications mentionnent l'utilisation, dans les procédés d'hydroisomérisation, de catalyseurs à base de zéolithes modifiées plus ou moins profondément, et en particulier de mordénites, usuellement sous forme acide, associées à au moins un métal du groupe VIII, de la classification périodique des éléments, qui apporte la fonction hydrogénante/déshydrogénante.

L'efficacité du catalyseur dépend en particulier de la bonne dispersion du métal sur la mordénite sous forme acide. Il est ainsi souhaitable d'avoir la plus grande dispersion possible du métal sur la mordénite, de manière à ce que le maximum d'atomes de métal soient accessibles aux réactifs. La taille des cristallites métalliques doit être faible, de préférence inférieure ou égale à $10 \times 10^{-10}$ m (10 Angstoms), et leur répartition au départ, sur le catalyseur fraichement préparé, (c'est-à-dire un catalyseur n'ayant pas été mis en contact avec des hydrocarbures dans des conditions d'isomérisation), doit être la plus homogène possible et surtout elle doit également être la plus homogène possible après la régénération du catalyseur au moins partiellement désactivé.

En effet le coke, qui se forme inévitablement lors de la réaction d'isomérisation, se dépose sur le catalyseur contribuant ainsi à la diminution des performances globales du catalyseur, qui doit être alors régénéré de façon à prolonger sa durée de vie totale.

La régénération classique du catalyseur comprend une étape dans laquelle on enlève le coke par combustion. Dans cette étape le catalyseur est chauffé dans un courant d'oxygène plus ou moins dilué à une température d'environ 400 à 500°C de manière à brûler le coke. Si des précautions particulières ne sont pas prises, lors de ce traitement, il se produit une perte plus ou moins importante de surface des particules de métal ce qui entraine évidemment une perte plus ou moins importante d'activité pour le catalyseur. Ce phénomène de frittage de la phase métallique est bien connu de l'homme du métier. C'est pourquoi des procédures spéciales de régénération de ces catalyseurs ont été mises au point.

Un mode particulier de régénération d'un catalyseur zéolithique contenant du platine est décrit dans le brevet US-A-3 986 982 ; cette régénération consiste à mettre le catalyseur, après brûlage du coke, en contact avec un mélange de gaz contenant un gaz inerte, de 0,5 à 20 % en volume d'oxygène et de 5 à 500 ppm en volume de chlore sous la forme de chlore, d'acide chlorhydrique ou d'un composé organique chloré ; puis à purger le catalyseur de manière enlever l'oxygène et le chlore résiduel, et enfin à réduire ce catalyseur sous courant d'hydrogène à une température de 200 à 600°C.

Cette méthode quoique fournissant une nette amélioration de l'activité catalytique ne permet pas une bonne redispersion du platine et donc ne permet pas de réobtenir une activité du catalyseur régénéré voisine de celle du catalyseur neuf.

La présente invention a pour objet un procédé de régénération d'un catalyseur contenant au moins un métal du groupe VIII supporté par une mordénite sous forme acide, qui a perdu au moins une partie de son activité initiale comprenant :

a) une étape d'enlèvement de la majeure partie du coke qui s'est déposé sur le catalyseur durant la mise en contact dudit catalyseur avec la charge d'hydrocarbures dans des conditions d'isomérisation et

b) une étape d'oxychloration du produit résultant de l'étape a) ladite oxychloration étant effectuée à une température d'environ 200 à 500°C à l'aide d'un mélange gazeux contenant de l'oxygène, de l'eau, et du chlore et/ou au moins un composé chloré.

Le catalyseur régénéré selon le procédé de la présente invention retrouve pratiquement son activité initiale.

La présente invention a également pour objet un procédé d'isomérisation comprenant :

1) une première étape dans laquelle le catalyseur d'isomérisation, contenant au moins un métal du groupe VIII supporté par une mordénite sous forme acide, est mis en contact avec la charge d'hydrocarbures, riche en paraffines normales ayant par exemple 4, 5, 6 ou 7 atomes de carbone par

2

molécule, et en particulier 5 ou 6 atomes de carbone par molécule, dans des conditions d'isomérisation, ladite étape étant poursuivie jusqu'à ce que le catalyseur ait perdu, au moins en partie, son activité initiale,

2) une deuxième étape dans laquelle le catalyseur (issu de la première étape) partiellement désactivé est traité par un gaz contenant de l'oxygène, de manière à brûler au moins en partie le coke qui s'est déposé sur le catalyseur au cours de la première étape,

3) une troisième étape dans laquelle le catalyseur, ne contenant plus de coke ou seulement une faible proportion de coke, est soumis à une oxychloration dans les conditions énoncées ci-dessus pour l'étape b) du procédé de régénération selon l'invention,

4) une quatrième étape dans laquelle le catalyseur issu de la troisième étape est au moins en partie réduit, de préférence en présence d'hydrogène et,

5) une cinquième étape dans laquelle le catalyseur, issu de la quatrième étape, est mis en contact avec une nouvelle charge d'hydrocarbures dans des conditions d'isomérisation.

Le cycle ainsi décrit comprenant une période d'isomérisation, une période de régénération, une période de réduction, puis une nouvelle période d'isomérisation peut être répété plusieurs fois.

Le procédé de régénération de la présente invention s'applique à un catalyseur à base d'une mordénite acide, ayant habituellement les caractéristiques données ci-après, supportant au moins un métal du groupe VIII, de préférence le platine, le palladium ou le nickel, ayant perdu, au moins en partie, son activité initiale.

La régénération du catalyseur est effectuée usuellement lorsque l'activité du catalyseur, dans les conditions choisies initialement pour la réaction d'isomérisation, ne représente plus que 50 à 90 % de l'activité initiale et de préférence 50 à 80 % de l'activité initiale. Bien que le procédé de la présente invention soit également applicable à des catalyseurs encore plus profondément désactivés, c'est-à-dire dont l'activité est inférieure à 50 % de l'activité initiale il est habituellement souhaitable, pour des raisons économiques, de régénérer le catalyseur avant que son activité ne devienne trop faible. Selon les conditions industrielles du site où l'isomérisation est effectuée l'homme du métier est apte à décider à quel moment la régénération doit être de préférence effectuée.

La mordénite est un alumino-silicate naturel ou synthétique caractérisé par un rapport atomique Si/Al compris généralement entre 4 et 6 ; sa structure cristalline est constituée d'enchaînements de tétraèdres de base $SiO_4$ et $AlO_4$ générateurs de deux types de canaux : des canaux à ouverture dodécagonale (contour à 12 oxygènes) et des canaux à ouverture octogonale (contour à 8 oxygènes).

La mordénite utilisée comme "base" du catalyseur employé dans le procédé d'isomérisation de n-paraffines selon la présente invention peut être également une mordénite désaluminée suivant les méthodes connues de l'homme du métier. Le rapport atomique Si/Al de la mordénite utilisée est habituellement d'environ 5 à environ 50, bien que des mordénites ayant un rapport atomique Si/Al plus élevé, par exemple jusqu'à 80 ou même plus, soient également utilisables.

On emploie de préférence une mordénite ayant un rapport atomique Si/Al de 5 à 30.

La mordénite utilisée peut être une mordénite dite à larges pores, de morphologie sphérulite, toujours synthétique ou une mordénite dite à petits pores, de morphologie en forme d'aiguilles, ayant des canaux "débouchés" telle que celle décrite dans le brevet européen n° 196 965.

La mordénite utilisée comme base du catalyseur, peut habituellement adsorber des molécules de diamètre cinétique supérieur à environ $6,6 \times 10^{-10}$ m (6,6 Angstroms) telles que le benzène. La mordénite qu'elle soit synthétique ou naturelle est au départ sous forme sodique et contient habituellement entre 4 et 6,5 % en poids de sodium par rapport au poids de mordénite sèche ; il est ainsi nécessaire d'effectuer sa mise sous forme acide, par tout procédé connu de l'homme du métier permettant d'obtenir une mordénite sous forme acide ayant une teneur en sodium inférieure à environ 0,2 % en poids et de manière préférée inférieure à environ 0,1 % en poids par rapport au poids de mordénite sèche.

Par mordénite sous forme acide on désigne dans la présente invention une mordénite contenant moins d'environ 0,2 % poids et de préférence moins d'environ 0,1 % poids de cations alcalins et alcalino terreux exprimé en équivalent poids de sodium par rapport au poids de la mordénite sèche.

La mordénite utilisée a habituellement un volume de maille, V, de la maille élémentaire de 2,73 à 2,78 nanomètre cube ($nm^3$) et de préférence de 2,74 à 2,77 $nm^3$ et sa capacité d'adsorption de benzène est usuellement d'au moins 5 % et de préférence au moins 8 % poids par rapport au poids de mordénite sèche.

La mordénite ainsi définie est utilisée pour préparer le catalyseur, soit seule, soit mélangée intimement à une matrice, généralement amorphe, par exemple à une poudre humide d'un gel d'alumine. Le mélange est ensuite mis en forme, par exemple par extrusion au travers d'une filière.

La teneur en mordénite du support ainsi obtenu doit être supérieure à environ 40 % et de préférence supérieure à 60 % en poids. Cette teneur en mordénite est dans ce cas habituellement d'environ de 40 à 95 % en poids et de préférence d'environ 60 à 90 % en poids de l'ensemble mordénite et matrice. La mise en forme peut être réalisée avec d'autres matrices que l'alumine, telles que par exemple, la silice-alumine, les argiles naturelles (par exemple le kaolin ou la bentonite) et l'alumine-oxyde de bore, et avec une technique autre que l'extrusion telle que, par exemple le pastillage, la dragéification ou toute autre technique connue de l'homme du métier.

Le métal hydrogénant du groupe VIII est ensuite déposé sur ce support par tout procédé connu de l'homme du métier permettant le dépôt du métal sur la mordénite. Dans le cas du platine, on utilisera un procédé d'échange cationique avec un complexe tétrammine de platine : le métal se déposera alors pratiquement en totalité sur la mordénite. Il est également possible d'introduire le métal du groupe VIII, directement sur la mordénite avant son mélange éventuel avec une matrice.

L'utilisation de la technique d'échange cationique peut être appliquée pour déposer le métal soit sur la poudre de mordénite, soit sur un produit déjà mis en forme, avec ou sans cation compétiteur ammonium. Sur extrudés ou sur poudre le métal peut également être déposé par la technique dite de l'imprégnation à sec. Le produit séché est ensuite généralement calciné entre 300 et 600°C.

Le solide ainsi obtenu contient habituellement d'environ 0,05 à 10 % en poids de métal du groupe VIII. Dans le cas du platine et du palladium la teneur (en poids) est habituellement d'environ 0,05 à 1 % et de préférence d'environ 0,1 à 0,6 %. Dans le cas du nickel la teneur pondérale est habituellement d'environ 0,1 à 10 % et de préférence d'environ 0,2 à 5 %.

Ce solide est un catalyseur d'isomérisation utilisable dans le procédé selon la présente invention. La répartition des cristallites métalliques est cependant relativement peu homogène. Les tailles des cristallites ont été mesurées à l'aide d'un microscope électronique à haute résolution. Le solide ou catalyseur destiné à être observé, par microscopie électronique à transmission, est broyé dans un mortier en agate, puis mis en suspension dans de l'éthanol par ultrasons. Une goutte de cette suspension est ensuite déposée sur une grille de cuivre recouverte d'un mince film de carbone à trou. Après un bref séchage, l'échantillon est observé par la technique dite du champ clair. La taille des cristallites métalliques, observée sur le solide obtenu par le procédé décrit ci-dessus, est comprise entre et $30 \times 10^{-10}$ et $200 \times 10^{-10}$ m (30 et 200 Angstroms). Il est possible d'obtenir un catalyseur présentant une meilleure dispersion des particules métalliques en faisant subir à ce solide un traitement d'oxychloration dans les conditions définies ci-après.

Le traitement d'oxychloration comprend la mise en contact du solide avec du chlore et/ou au moins un composé chloré en présence d'un gaz contenant de l'oxygène et de la vapeur d'eau habituellement à une température d'environ 200 à 500°C et de préférence d'environ 300 à 500°C, le chlore et/ou le composé chloré étant utilisé en une quantité représentant au total de 0,5 à 10 % poids calculé en poids de chlore par rapport au poids de mordénite sèche, et de préférence de 1 à 5 % poids. Le composé chloré peut être un composé chloré minéral ou organique. Lorsqu'on utilise un composé organique chloré, on préfère habituellement employer un chloroalkane.

L'opération d'oxychloration du solide à base de mordénite peut être effectuée "hors site" ou "ex situ" ou encore "in situ" ou "en site".

Par traitement "in situ" on désigne un traitement qui est effectué en tête de la (ou des) zone(s) dans laquelle sera effectuée la réaction proprement dite d'isomérisation ou dans une (ou des) zone(s) plus ou moins en communication directe avec ladite zone d'isomérisation.

Par traitement "ex situ" on désigne un traitement effectué soit à proximité du site de l'unité industrielle d'isomérisation, dans une zone qui ne se trouve pas au voisinage immédiat de la zone d'isomérisation, soit à distance plus ou moins grande géographiquement de l'unité industrielle (là où le solide a été fabriqué par exemple).

Le traitement d'oxychloration consiste habituellement à chauffer le solide, comprenant au moins un métal du groupe VIII déposé sur la mordénite acide, en présence d'un courant gazeux contenant de l'oxygène et de la vapeur d'eau, par exemple de l'air humide, ou de l'oxygène dilué par un gaz inerte, la teneur en oxygène du mélange gazeux étant habituellement d'environ 10 à 50 % et de préférence d'environ 15 à 35 % en poids et sa teneur pondérale en eau étant habituellement d'environ 0,01 à 5 %, et de préférence d'environ 0,03 à 2% et de façon souvent avantageuse d'environ 0,05 à 1 %. Le chauffage en présence du mélange gazeux contenant de l'oxygène et de l'eau est généralement effectué de manière progressive jusqu'à la température choisie. La température augmente par exemple d'environ 5°C par minute jusqu'à la température choisie. On introduit alors dans le courant gazeux d'oxygène et de vapeur d'eau, maintenu à la température choisie , du chlore ($Cl_2$) ou un composé chloré par exemple, l'acide chlorhydrique (HCl) ou un composé chloré organique tel que le tétrachlorure de carbone, le dichloropropane, le dichloroéthane ou le chloroforme.

Le débit d'injection du chlore ou du composé chloré est habituellement calculé de manière à ce que la durée nécessaire à l'injection de la quantité choisie de chlore soit d'environ 0,5 à 6 heures et de préférence d'environ une heure et demie à environ 2 heures. Lorsque l'introduction du chlore est terminée le catalyseur est alors refroidi, en présence du courant gazeux contenant de l'oxygène et de la vapeur d'eau décrit ci-dessus, généralement jusqu'à la température ambiante.

La teneur en chlore résiduel sur le catalyseur n'excède habituellement pas environ 30 à 50% poids de la masse de chlore injecté. A cette teneur, le chlore n'est pas préjudiciable à la structure de la mordénite : c'est-à-dire que la structure de la mordénite n'est pas sensiblement modifiée.

La répartition des particules métalliques, sur le catalyseur obtenu après oxychloration, est homogène et la taille des cristallites métalliques n'excède pas $7 \times 10^{-10}$ m (7 Angstroms).

La première étape du procédé de régénération selon l'invention doit être effectuée de préférence sur un catalyseur ne contenant pratiquement pas d'eau. Si le catalyseur que l'on veut régénérer provient d'une zone de stockage dans laquelle le catalyseur, au moins partiellement désactivé, a été accumulé, il est préférable avant d'effectuer la combustion du coke par l'oxygène de chauffer le catalyseur sous gaz inerte à une température d'environ 150°C pendant un temps suffisant pour bien éliminer l'eau, avant de monter en température au cours de l'étape de combustion du coke. Ce séchage permet d'éviter une désalumination plus poussée de la mordénite, désalumination qui risque de se produire lorsque le catalyseur contient de l'eau, lors de la combustion du coke, si malgré les précautions prises la température atteint ou dépasse 500°C. Le séchage est effectué pendant un temps suffisant pour que la teneur en eau soit inférieure à environ 100 ppm en poids et de préférence inférieure à environ 50 ppm en poids par rapport au poids du catalyseur. Le terme gaz inerte désigne dans la présente demande tout gaz qui ne réagit pas avec le catalyseur, tel que par exemple l'azote, l'hélium et l'argon ou des mélanges de ces gaz.

La première étape d'élimination de la majorité du coke contenu sur le catalyseur, est effectuée par mise en contact du catalyseur avec un gaz contenant de l'oxygène en augmentant progressivement la température jusqu'à ce que l'on observe la réaction exothermique de combustion ou brûlage du coke, habituellement entre 300 et 500°C. Cette combustion est effectuée avec précautions et les conditions opératoires sont ajustées de manière à ce que de préférence la température ne dépasse pas 550°C et d'une manière la plus préférée ne dépasse pas 500°C. Au cours de cette étape de combustion la majorité du coke est brûlé de manière à ce que la teneur pondérale en coke résiduel du catalyseur après combustion soit généralement inférieure à environ 20 % et de préférence inférieure à environ 10 % de la teneur en coke du catalyseur avant combustion (c'est-à-dire que au moins 80 % et de préférence au moins 90 % du coke a été brûlé). Le gaz contenant de l'oxygène, dans l'étape de combustion est habituellement un mélange d'oxygène et de gaz inerte, contenant habituellement de 0,1 à 30 % en poids d'oxygène et de préférence de 0,2 à 10 % en poids d'oxygène, ce peut être par exemple de l'air ou de l'air dilué par un gaz inerte. La proportion d'oxygène dans le gaz employé pour la combustion du coke peut également être variable en fonction de l'évolution de la réaction exothermique de combustion.

Dans une forme préférée de réalisation de l'étape de combustion du coke le courant gazeux contenant de l'oxygène que l'on met en contact avec le catalyseur contiendra en outre du chlore et/ou au moins un composé chloré. L'emploi de chlore et/ou de composé chloré permet essentiellement de mieux maintenir l'acidité du support et de réduire le phénomène de frittage de la phase métallique. Le chlore et/ou le composé chloré tel que par exemple l'acide chlorhydrique (HCl) ou un composé chloré organique tel que le tétrachlorure de carbone, le dichloropropane, le dichloroéthane ou le chloroforme est utilisé en une quantité représentant au total de 0,1 à 5 % et de préférence de 0,2 à 3 % en poids calculé en poids de chlore par rapport au poids de mordénite sèche contenu dans le catalyseur.

Le catalyseur après brûlage du coke est de préférence mis sous gaz inerte, puis soumis à la deuxième étape du procédé de régénération selon l'invention éventuellement après avoir ajusté sa température par exemple à la valeur souhaitée pour l'oxychloration. Il est également possible de refroidir le catalyseur après brûlage du coke jusqu'à la température ordinaire et de la maintenir sous gaz inerte à cette température, avant de le soumettre à l'oxychloration ce sera par exemple le cas si le brûlage du coke et l'oxychloration ne sont pas effectués sur le même site.

Malgré les précautions prises pour limiter au maximum le phénomène de frittage de la phase métallique, lors de la combustion du coke la taille des particules métalliques que l'on peut observer par microscopie électronique a considérablement augmenté. Dans le cas où le catalyseur fraîchement préparé avait des tailles de particules inférieures à environ $7 \times 10^{-10}$ m (7 Angstroms) on observe après brûlage du coke des particules de tailles comprises entre environ $10 \times 10^{-10}$ m et environ $50 \times 10^{-10}$ m. Dans le cas où le catalyseur fraîchement préparé avait des tailles de particules comprises entre environ $30 \times 10^{-10}$ m et environ $200 \times 10^{-10}$ m, on observe après brûlage du coke des particules de très grosse taille pouvant atteindre $2000 \times 10^{-10}$ m

Le catalyseur issu de l'étape a) de combustion du coke est soumis à une oxychloration dans des conditions identiques à celles décrites ci-avant pour l'oxychloration du solide à base de mordénite sous forme acide contenant au moins un métal du groupe VIII.

Ce traitement d'oxychloration est généralement effectué après l'étape de combustion du coke. Bien qu'il soit possible également d'effectuer la combustion et l'oxychloration de façon simultanée, on préfère généralement effectuer d'abord la combustion du coke, puis le traitement d'oxychloration.

Dans les deux cas mentionnés ci-dessus on observe après l'étape d'oxychloration une répartition très homogène des particules métalliques sur le catalyseur, et la taille des particules métalliques n'excède pas $7 \times 10^{-10}$ m.

Après le traitement d'oxychloration, le catalyseur régénéré obtenu est, de préférence, soumis à une réduction avant d'être mis en contact avec une nouvelle charge d'hydrocarbures dans des conditions d'isomérisation. La réduction est généralement effectuée à l'aide d'un gaz contenant au moins un composé réducteur, de préférence l'hydrogène. On peut utiliser de l'hydrogène dilué par un gaz inerte, de l'hydrogène industriel ou de l'hydrogène essentiellement pur, c'est-à-dire contenant moins de 0,5 % en volume et de préférence moins de 0,1 % en volume d'impuretés. La réduction est habituellement effectuée par paliers jusqu'à une température de 350 à 750°C et de préférence de 400 à 600°C pendant un temps suffisant pour que les concentrations en composés réducteurs soient les mêmes à l'entrée et à la sortie du réacteur, ce qui prouve que la réduction dans les conditions choisies est terminée. Cette étape de réduction est de préférence réalisée "in situ".

Dans l'étape d'isomérisation, la charge riche en paraffines légères à 5 ou 6 atomes de carbone et l'hydrogène sont mis en contact avec un catalyseur frais ou régénéré décrit ci-dessus dans les conditions de l'isomérisation. Ce contact peut s'effectuer en utilisant le catalyseur en lit fixe, en lit fluidisé ou en batch (c'est-à-dire en discontinu).

L'étape d'isomérisation du procédé selon l'invention est usuellement effectuée à une température de 200 à 350°C et de préférence de 230 à 300°C à des pressions partielles d'$H_2$ comprises entre la pression atmosphérique (0,1 MPa) et 7 MPa et de préférence entre 0,5 MPa et 5 MPa. La vitesse spatiale peut être comprise entre 0,1 et 20 litres d'hydrocarbures liquides par litre de catalyseur et par heure et de préférence

0 283 343

entre 1 et 10. Le rapport molaire $H_2$/charge peut varier entre de larges limites et est compris normalement entre 0,2 et 20 et de préférence entre 0,5 et 10. L'isomérisation étant une réaction équilibrée, l'isomérisat contient encore une quantité importante de n-paraffines non converties. Ces paraffines peuvent être séparées des isomères par exemple par distillation ou par fractionnement sur tamis moléculaire et recyclées dans l'unité d'isomérisation.

La coupe d'hydrocarbures utilisée contient généralement au moins 80 % poids et de préférence au moins 90 % poids de n-paraffines à 4, 5, 6 ou 7 atomes de carbone.

Les coupes d'hydrocarbures qui sont préférentiellement traitées sont celles qui contiennent au moins 80 % en poids d'hydrocarbures à 5 et/ou 6 atomes de carbone et mieux au moins 90 % en poids desdits hydrocarbures.

L'équilibre thermodynamique entre les différents isomères varie énormément avec la température. Les hydrocarbures ramifiés, qui sont ceux qui ont un indice d'octane élevé sont d'autant plus favorisés que la température est plus basse. Le problème de l'isomérisation des paraffines consiste donc à trouver des catalyseurs actifs à la plus basse température possible. Le nombre de moles de diméthyl-2,2-butane (22 DMC4), dont l'indice d'octane est l'un des plus élevé parmi les isomères en C6, ne peut dépasser, à une température donnée, sa valeur à l'équilibre thermodynamique : Chimie des hydrocarbures par G. Lefebvre, éditions Technip 1978, pages 89 à 91 et US-A-4238319 (colonne 1 lignes 20 à 66). L'approche à l'équilibre en pourcent pour le 22DMC4 définie comme le nombre de moles de 22DMC4 dans l'effluent de la réaction (encore appelée la recette) multiplié par cent et divisé par le nombre de moles de 22DMC4 à l'équilibre thermodynamique, à la température considérée, est une mesure permettant de comparer facilement les activités relatives des catalyseurs.

La présente invention concerne également un prétraitement préalable du catalyseur d'isomérisation selon une méthode inspirée du procédé de régénération décrit ci-dessus. Ce prétraitement permet notamment d'éviter les inconvénients qui surviennent ultérieurement après une régénération classique des catalyseurs. En outre ce prétraitement, lorsqu'il est effectué en combinaison avec la régénération selon l'invention, précédemment décrite, permet l'obtention d'excellents résultats.

Le procédé est caractérisé en ce que :

(a) On met en contact ledit catalyseur avec un gaz contenant au moins de l'oxygène moléculaire et 0,01 à 5 % en poids d'eau à une température que l'on élève progressivement jusqu'à ce qu'elle se trouve comprise entre 200 et 550°C, de préférence entre 300 et 550°C (et plus particulièrement entre 350 et 400°C).

(b) On poursuit l'addition dudit gaz, à une température comprise entre 200 et 500°C (de préférence entre 300 et 500°C), en y ajoutant du chlore ou un composé chloré de façon à introduire au total, dans le catalyseur 0,5 à 10 % en poids de chlore (de préférence 1 à 5 %) par rapport au poids de la mordénite contenue dans le catalyseur.

(c) On réduit le catalyseur obtenu à l'étape précédente à l'aide d'un gaz contenant au moins un composé réducteur (de l'hydrogène par exemple) à une température comprise entre 300 et 750°C pendant un temps suffisant pour que la concentration en composé réducteur soit sensiblement la même à l'entrée et à la sortie de la zone où s'effectue la réduction du catalyseur.

On effectue ensuite la réaction d'isomérisation ; pour ce, par exemple, on traite en présence du catalyseur réduit à l'étape précédente, ladite coupe d'hydrocarbures, en présence d'hydrogène, à une température comprise entre 200 et 350°C environ sous une pression partielle d'hydrogène comprise entre 0,1 et 7 MPa, avec une vitesse spatiale horaire de la charge d'hydrocarbures comprise entre 0,1 et 20 $h^{-1}$ et un rapport molaire $H_2$/charge d'hydrocarbures compris entre 0,2 et 20.

Le composé chloré utilisé à l'étape (b) est choisi dans le groupe constitué par l'acide chlorhydrique, le tétrachlorure de carbone, le dichloropropane, le chloroforme et le dichloroéthane.

Les étapes (a) et (b) du procédé selon l'invention peuvent être effectuées dans deux zones distinctes ou dans une même zone. Autrement dit, l'étape (a) peut être effectuée "hors site" (ou "ex situ") ou encore "in situ" ou "en site".

Le prétraitement consiste généralement à chauffer le solide, comprenant au moins un métal du groupe VIII déposé sur la mordénite acide, en présence d'un courant gazeux contenant de l'oxygène et de la vapeur d'eau, par exemple de l'air humide, ou de l'oxygène dilué par un gaz inerte, la teneur en oxygène du mélange gazeux étant généralement de 10 à 50 % et de préférence de 15 à 35 % en poids et sa teneur pondérale en eau étant généralement de 0,01 à 5 %, et de préférence de 0,05 à 1 %. Le chauffage en présence du mélange gazeux contenant de l'oxygène et de l'eau est généralement effectué de manière progressive jusqu'à la température choisie. La température augmente par exemple d'environ 5°C par minute jusqu'à la température choisie. On introduit alors dans le courant gazeux d'oxygène et de vapeur d'eau, maintenu à la température choisie , du chlore ($Cl_2$) ou un composé chloré par exemple, l'acide chlorhydrique (HCl) ou un composé chloré organique tel que le tétrachlorure de carbone, le dichloropropane, le dichloroéthane ou le chloroforme.

Le débit d'injection du chlore ou du composé chloré est généralement calculé de manière à ce que la durée nécessaire à l'injection de la quantité choisie de chlore soit d'environ une heure et demie à environ 2 heures. Lorsque l'introduction du chlore est terminée le catalyseur est alors refroidi, en présence du courant gazeux contenant de l'oxygène et de la vapeur d'eau décrit ci-dessus, généralement jusqu'à la température ambiante.

La teneur en chlore résiduel sur le catalyseur n'excède généralement pas environ 30 à 50% poids de la masse de chlore injecté. A cette teneur, le chlore n'est pas préjudiciable à la structure de la mordénite :

6

c'est-à-dire que la structure de la mordénite n'est pas sensiblement modifiée.

La répartition des particules métalliques, sur le catalyseur obtenu après oxychloration, est homogène et la taille des cristallites métalliques n'excède pas $7 \times 10^{-10}$ m (7 Angstroms).

Dans le prétraitement, après le traitement par du chlore ou un composé chloré, le catalyseur obtenu est soumis à une réduction avant d'être mis en contact avec la charge d'hydrocarbures dans des conditions d'isomérisation. La réduction est généralement effectuée à l'aide d'un gaz contenant au moins un composé réducteur, de préférence l'hydrogène. On peut utiliser de l'hydrogène dilué par un gaz inerte ou de l'hydrogène essentiellement pur, c'est-à-dire contenant moins de 0,5 % en volume et de préférence moins de 0,1 % en volume d'impuretés. La réduction est généralement effectuée par paliers jusqu'à une température de 350 à 750°C et de préférence de 400 à 600°C pendant un temps suffisant pour que les concentrations en composés réducteurs soient les mêmes à l'entrée et à la sortie du réacteur, ce qui prouve que la réduction dans les conditions choisies est terminée. Cette étape de réduction est de préférence réalisée "in situ".

Les exemples suivants définissent l'invention sans en limiter la portée.

Les performances des catalyseurs sont définies par la conversion (C) du n-hexane, la sélectivité (S) en isomérisation, l'approche à l'équilibre (Ap) sur le diméthyl-2- 2-butane et par l'indice d'octane recherche (10).

$$\text{Conversion (C \%)} = \frac{\text{(Masse de n-hexane entrée-masse de n-hexane sortie)}}{\text{Masse de n-hexane entrée}} \times 100$$

$$\text{Sélectivités (S\%)} = \frac{\text{(somme masse des isomères)} \times 100}{\text{(somme masse des produits de la réaction)}}$$

$$\text{Approche à l'équilibre (Ap\%)} = \frac{\text{(nombre de moles de 22DMC4 dans la recette)} \times 100}{\text{nombre de moles de 22DMC4 à l'équilibre.}}$$

## EXEMPLE N° 1

La matière première est une mordénite petits pores référence Alite 150 de la Société Chimique de la Grande Paroisse. Sa formule chimique à l'état anhydre est : Na $AlO_2(SiO_2)_{5,5}$, et sa capacité d'adsorption de benzène est de 1 % en poids par rapport au poids de solide sec (volume de maille : 2,79 nm³ ; teneur en sodium 5,3 % (poids), diamètre cinétique des molécules adsorbées : 3,8 × $10^{-10}$ m) ; 50 g de cette poudre sont plongés dans une solution 2M de nitrate d'ammonium et la suspension est portée à 95°C pendant deux heures.

Le volume de la solution de nitrate d'ammonium engagée est égal à 4 fois le poids de zéolithe sec (V/P = 4). Cette opération d'échange cationique est recommencée 3 fois. Après le 3ème échange, le produit est lavé à l'eau, à 20°C pendant 20 minutes avec un rapport V/P égal à 4. La teneur en sodium, exprimée en pourcentage en poids par rapport au poids sec passe de 5,3 à 0,1 %. Le produit est ensuite filtré et soumis à une calcination en atmosphère confinée (self steaming) à 600°C pendant 2 heures.

On procède ensuite à une attaque acide avec de l'acide chlorhydrique 0,58N, en portant le produit à reflux dans la solution aqueuse d'acide chlorhydrique à 90°C pendant 2 heures avec un rapport V/P égal à 8. Le produit est ensuite filtré, lavé à l'acide chlorhydrique 0,1N puis à l'eau.

Le rapport atomique Si/Al de cette mordénite est égal à 12, son volume de maille à 2,750 nm³, son taux de sodium à 300 ppm et sa capacité d'adsorption de benzène à 9,6 % poids par rapport au poids de solide sec. La morphologie de cette mordénite est en forme d'aiguilles de longueurs moyennes 5 × $10^{-6}$m dont les faces sont hexagonales et ont une longueur d'environ 1 × $10^{-6}$m et une hauteur d'environ 0,3 × $10^{-6}$m. Cette mordénite ainsi modifiée est ensuite malaxée avec un liant de type aluminique, puis ce mélange, contenant 25 % poids d'alumine est forcé au travers d'une filière. Les extrudés de diamètre 1,2 × $10^{-3}$m sont ensuite séchés et calcinés.

0,4 % de platine sont ensuite déposés sur ce support par échange cationique à partir de chlorure de platine tétrammine $Pt(NH_3)_4Cl_2$ avec du nitrate d'ammonium comme ion compétiteur. La quantité de sodium dans le catalyseur final est égal à 80 ppm. Le rapport atomique Si/Al est égal à 12 et le volume de maille à 2,750 nm³. Les extrudés sont ensuite séchés puis calcinés à 500°C. La répartition des particules métalliques est relativement hétérogène et leur taille comprise entre $10 \times 10^{-10}$m et $200 \times 10^{-10}$m (10 A et 200 A). On appelle A ce catalyseur ainsi obtenu :

Le catalyseur A ainsi obtenu est chargé dans une unité catalytique en lit fixe et réduit sous hydrogène par paliers de 2 heures à 150, 250, 350 et 450°C. Il est ensuite testé avec une charge de normal hexane dans les conditions suivantes : température 250°C, pression 30 bars, poids de n-hexane par unité de poids de mordénite et par heure : 2, rapport molaire hydrogène sur normal hexane : 2. Les performances indiquées dans le tableau I sont prises après 30 h de mise en régime du catalyseur, puis après 12 mois de fonctionnement. Au bout de 12 mois de fonctionnement on constate que les performances ont diminué d'environ 50 %, la teneur en coke du catalyseur est de l'ordre de 5 % en poids. Le catalyseur A est alors soumis à un traitement de régénération selon le processus suivant (non conforme à celui de la présente invention) :

on introduit dans le réacteur de régénération, contenant le catalyseur A, sous pression atmosphérique, un mélange gazeux contenant de l'air sec, de l'azote et du chlore sous forme de tétrachlorure de carbone. Le mélange contient 4 % en poids d'oxygène et 0,3 % en poids de chlore. La température est augmentée progressivement jusqu'au démarrage de la combustion du coke et on régule ensuite le débit de mélange gazeux de manière à ce que la température à laquelle le coke brûle reste aux environs de 380°C. L'injection du mélange gazeux est poursuivie jusqu'à ce que la température redescende à 300°C, on refroidit alors le catalyseur sous azote. Le catalyseur obtenu est appelé catalyseur A', sa teneur en coke est de l'ordre de 0,5 % en poids et son rapport Si/Al de charpente est toujours égal à 12. Les particules observées en microscopie électronique ont une taille allant jusqu'à $2000 \times 10^{-10}$m.

Le catalyseur A' est chargé dans une unité catalytique en lit fixe et réduit sous hydrogène par palier de 2 heures à 150, 250, 350 et 450°C. Il est ensuite testé dans les mêmes conditions que celles énoncées ci-dessus pour le catalyseur A.

Les performances du catalyseur A' indiquées dans le tableau I sont prises après 30 h de mise en régime du catalyseur. On constate que le catalyseur A' n'a pas retrouvé le niveau d'activité du catalyseur A frais, mais a regagné un peu d'activité par rapport au catalyseur A ayant travaillé 12 mois.

**EXEMPLE 2.**

Un autre test est effectué avec une nouvelle charge de catalyseur A dans les mêmes conditions que celles décrites dans l'exemple 1. Au bout de 12 mois de fonctionnement le catalyseur A, est soumis à un traitement de régénération suivant un protocole conforme à l'invention effectué comme suit :
- montée en température depuis la température ordinaire jusqu'à 400°C en une heure et demie sous un courant d'air contenant de l'ordre de 1000 ppm en poids d'eau (0,1%),
- injection de chlore, dans l'air humide maintenu à 400°C sous forme de tétrachlorure de carbone, de manière à introduire 2 % en poids de chlore par rapport au poids de la mordénite contenue dans le catalyseur en une heure et demie,
- descente progressive jusqu'à la température ambiante sous courant d'air humide (1000 ppm en poids d'eau).

Le catalyseur obtenu est appelé catalyseur B ; ce catalyseur B est un catalyseur A régénéré selon le procédé de l'invention.

Une analyse du catalyseur B en microscopie électronique montre que toutes les particules métalliques ont une taille inférieure à $7 \times 10^{-10}$m. La structure de la mordénite n'a pas été modifiée par le taitement d'oxychloration. La teneur en chlore du catalyseur est de l'ordre de 0,6 % poids.

Le catalyseur B est réduit sous hydrogène par paliers de 2 heures à 150, 250, 350 et 450°C. Il est ensuite testé dans les mêmes conditions que celles énoncées dans l'exemple 1 pour le catalyseur A.

Les performances du catalyseur B indiquées dans le tableau I sont prises après 30 h de mise en régime du catalyseur. On constate que le catalyseur B a retrouvé le niveau d'activité du catalyseur A frais, il est de plus meilleur au niveau de la conversion et de la production du 22DMC4 que le catalyseur A frais.

**EXEMPLE 3**

On prépare un catalyseur C à partir du catalyseur A fraîchement préparé suivant le protocole décrit dans l'exemple 1.

Le catalyseur A est chargé dans l'unité catalytique puis avant d'etre utilisé est d'abord soumis à un prétraitement effectué comme suit :
- montée en température depuis la température ordinaire jusqu'à 400°C en une heure et demie sous un courant d'air humide contenant de l'ordre de 1000 ppm en poids d'eau,
- injection de chlore, dans l'air humide maintenu à 400°C sous forme de tétrachlorure de carbone, de manière à introduire 2 % en poids de chlore par rapport au poids de la mordénite contenu dans le catalyseur en une heure et demie,
- descente progressive jusqu'à la température ambiante sous courant d'air humide (1000 ppm en poids d'eau).

Le catalyseur obtenu est appelé catalyseur C, il est réduit sous hydrogène par palier de 2 heures à 150, 250, 350 et 450°C, puis testé dans les mêmes conditions que celles énoncées pour le catalyseur A dans l'exemple 1.

Les performances du catalyseur C sont mentionnées dans le tableau II après 30 heures de mise en régime, puis après 12 mois de fonctionnement.

0 283 343

Au bout des 12 mois de fonctionnement les performances du catalyseur C ont très nettement moins diminué par rapport à celles du catalyseur A non prétraité. Plus exactement, le catalyseur C possède le niveau d'activité du Catalyseur A mais il est bien meilleur au niveau de la conversion et de la production du 22DMC4 que le Catalyseur A.

Le catalyseur C est alors néanmoins régénéré selon le processus décrit dans l'exemple 1 pour la régénération du catalyseur A non conforme à l'invention. Le catalyseur régénéré C est appelé catalyseur C'. Le catalyseur C' est testé après réduction sous hydrogène par paliers de 2 heures à 150, 250, 350 et 450°C dans les mêmes conditions que celles énoncées dans l'exemple 1 pour le catalyseur A. Les performances du catalyseur C' après 30 heures de mise en régime figurent dans le tableau II. La taille des particules métalliques du catalyseur C' varie de $20 \times 10^{-10}$m à $200 \times 10^{-10}$m.

Un autre test est effectué avec une nouvelle charge de catalyseur C dans les mêmes conditions que celles décrites dans l'exemple 3. Au bout de 12 mois de fonctionnement le catalyseur C est soumis à un traitement de régénération suivant le protocole décrit dans l'exemple 3. Le catalyseur C' obtenu est chargé dans une unité catalytique en lit fixe puis oxychloré suivant le protocole décrit dans l'exemple 2 pour l'oxychloration du catalyseur A'. Le catalyseur obtenu après oxychloration est appelé catalyseur D. La taille des particules métalliques du catalyseur D est inférieure à $7 \times 10^{-10}$ m.

Le catalyseur D est réduit, puis testé dans les conditions énoncées dans l'exemple 2 pour le test du catalyseur B. Les performances du catalyseur D, après 30 h de mise en régime, sont mentionnées dans le tableau II.

On constate que le catalyseur C' de comparaison ne retrouve pas les performances du catalyseur C frais et que le catalyseur D régénéré selon le procédé de l'invention donne des performances légèrement meilleures que celles du catalyseur C frais.

## TABLEAU I.

| CATALYSEUR | | CONV. % | SELECT. % | Ap % 22DMC4 | IO |
|---|---|---|---|---|---|
| A | 30 h | 79,5 | 98,6 | 54,7 | 67 |
| | 12 mois | 35 | 98,6 | 25 | 60 |
| A' (régénéré) | 30 h | 66 | 98,8 | 31,7 | 63 |
| B (régénéré*) | 30 h | 81,2 | 98,2 | 67,0 | 69,6 |
| | 12 mois | 79,5 | 98,5 | 54,2 | 65,1 |

* selon l'invention.

9

## TABLEAU II.

| CATALYSEUR | | CONV. % | SELECT % | Ap %<br>22DMC4 | IO |
|---|---|---|---|---|---|
| C | 30 h | 81 | 98,0 | 67,0 | 69,6 |
| | 12 mois | 77,4 | 97,9 | 65 | 66,1 |
| C' | 30 h | 79 | 98,0 | 66,8 | 69,5 |
| D | 30 h | 81,3 | 98, 3 | 67,2 | 69,6 |
| | 12 mois | 79,7 | 98,6 | 54,9 | 66,2 |

C : Catalyseur A activé ; C' : activé et régénéré ; D : activé et régénéré selon l'invention.

**EXEMPLE 4**

Dans cet exemple, on régénère, au bout de 12 mois, le catalyseur A, dans les conditions selon l'invention décrites dans l'exemple 2.

Toutefois, la teneur en eau dans l'air utilisée pour l'étape d'oxychloration varie. Elle est respectivement de 50 ppm, 250 ppm, 1000 ppm, 1,5%, 3% pour les catalyseurs B1, B2, B (conforme à l'exemple 2), B3 et B4. Ces différents catalyseurs sont ensuite réduits sous hydrogène par paliers de 2 heures à 150°C, 250°C, 350°C et 450°C. Ils sont ensuite testés dans les mêmes conditions que celles énoncées dans l'exemple 1 pour le catalyseur A. Les performances de ces catalyseurs après 30 heures de fonctionnement du catalyseur figurent dans le tableau III ci-après.

TABLEAU III

| CATALYSEUR | Teneur en Eau (ppm) | Conv. % | Select. % | App % 22DMC4 | I O |
|------------|------|------|------|------|------|
| B1 | 50 | 66 | 98,8 | 31,7 | 63 |
| B2 | 250 | 79,1 | 98,6 | 55,2 | 67,2 |
| B | 1000 | 81,2 | 98,2 | 67,0 | 69,6 |
| B3 | 15000 (1,5%) | 78 | 98,5 | 54,2 | 67,0 |
| B4 | 30000 (3%) | 60 | 96,0 | 29,8 | 61, |

**Revendications**

1.- Procédé de régénération d'un catalyseur d'isomérisation, renfermant au moins un métal du groupe VIII, de la classification périodique des éléments, supporté par une mordénite sous forme acide caractérisé en ce qu'il comprend :

a) une première étape d'élimination de la majorité du coke, contenu sur ledit catalyseur, par combustion à l'aide d'un gaz contenant de l'oxygène à une température contrôlée inférieure à 550° C et,

b) une deuxième étape dans laquelle le produit issu de l'étape a) est oxychloré à une température d'environ 200 à 500° C à l'aide d'un mélange gazeux contenant de l'oxygène, de l'eau et au moins un agent choisi dans le groupe constitué par le chlore et les composés chlorés, la quantité de chlore et/ou de composé chloré employée représentant au total de 0,5 à 10 % en poids calculé en poids de chlore par rapport au poids de mordénite sèche.

2.- Procédé selon la révendication 1 dans lequel la première étape d'élimination du coke est conduite de manière à ce que la température reste inférieure à environ 500° C et à ce que la teneur pondérale du coke résiduel sur le catalyseur après la combustion, soit inférieure à 20 % de la teneur pondérale en coke du catalyseur avant combustion.

3.- Procédé selon la revendication 1 ou 2 dans lequel le gaz employé dans l'étape a) pour la combustion du coke contient de 0,1 à 30 % en poids d'oxygène.

4.- Procédé selon l'une des revendications 1 à 3 dans lequel le gaz employé dans l'étape a) pour la combustion du coke contient en outre du chlore et/ou au moins un composé chloré, la quantité de chlore et/ou de composé chloré employée représentant au total de 0,1 à 5 % en poids calculé en poids de chlore par rapport au poids de mordénite sèche.

5.- Procédé selon l'une des revendications 1 à 4 dans lequel le mélange gazeux employé dans l'étape b) pour l'oxychloration est de l'air humide additionné de chlore et/ou d'au moins un composé chloré.

6.- Procédé selon l'une des revendications 1 à 5 dans lequel le mélange gazeux employé dans l'étape b)

11

pour l'oxychloration contient de 0,01 à 5 % en poids d'eau (100 à 50.000 ppm).

7.- Procédé selon l'une des revendications 1 à 6 dans lequel le catalyseur d'isomérisation renferme au moins un métal du groupe VIII choisi dans le groupe formé par le platine, le palladium et le nickel, supporté par une mordénite sous forme acide ayant un rapport atomique Si/Al d'environ 5 à environ 50, et en ce que la mordénite sous forme acide est une mordénite ayant un volume V de la maille élémentaire de 2,73 à 2,78 $nm^3$, une capacité d'adsorption de benzène supérieure à 5 % poids par rapport au poids de mordénite sèche et adsorbant des molécules de diamètre cinétique supérieur à environ $6,6 \times 10^{-10}$m.

8.- Procédé d'isomérisation d'une coupe d'hydrocarbures, riche en n-paraffines ayant de 4 à 7 atomes de carbone par molécule, comprenant

- une période de mise en contact de ladite coupe dans des conditions d'isomérisation avec un catalyseur d'isomérisation contenant au moins un métal du groupe VIII supporté par une mordénite sous forme acide, ledit contact étant poursuivi jusqu'à ce que le catalyseur ait perdu au moins en partie son activité initiale,

- une période b) de régénération du catalyseur issu de la première période a) ladite régénération étant effectuée selon le procédé de l'une des revendications 1 à 7 et,

- une période c) dans laquelle le catalyseur régénéré issu de la période b) est au moins en partie réduit puis renvoyé à la période a) de mise en contact avec une coupe d'hydrocarbures.

9.- Procédé d'isomérisation selon la revendication 7, caractérisé en ce que :

(a) on met en contact le catalyseur avec un gaz contenant au moins de l'oxygène moléculaire et 0,01 à 5 % en poids d'eau à une température que l'on élève progressivement jusqu'à ce qu'elle se trouve comprise entre 200 et 550°C,

(b) on poursuit l'addition dudit gaz, à une température comprise entre 200 et 500°C, en y ajoutant du chlore ou un composé chloré de façon à introduire au total, dans le catalyseur, 0,5 à 10 % en poids de chlore par rapport au poids de la mordénite contenue dans le catalyseur,

(c) on réduit le catalyseur obtenu à l'étape précédente à l'aide d'un gaz contenant au moins un composé réducteur, à une température comprise entre 300 et 750°C pendant un temps suffisant pour que la concentration en composé réducteur soit sensiblement la même à l'entrée et à la sortie de la zone où s'effectue la réduction du catalyseur,

(d) on traite en présence du catalyseur réduit à l'étape précédente, ladite coupe d'hydrocarbures, en présence d'hydrogène, à une température comprise entre 200 et 350°C environ, sous une pression partielle d'hydrogène comprise entre 0,1 et 7 MPa, avec une vitesse spatiale horaire de la charge d'hydrocarbures comprise entre 0,1 et 20 $h^{-1}$ et un rapport molaire $H_2$/charge d'hydrocarbures compris entre 0,2 et 20.

10.- Procédé selon la revendication 9, dans lequel l'étape (a) et l'étape (b) sont effectuées dans deux zones distinctes.

11.- Procédé selon l'une des revendications 9 et 10, dans lequel l'étape (a) et l'étape (b) sont effectuées dans une même zone.

12.- Procédé selon l'une des revendications 9 à 11 dans lequel, à l'étape (a), on chauffe jusqu'à une température comprise entre 350 et 400°C et dans lequel à l'étape (b), on ajoute du chlore ou un composé chloré de façon à introduire dans le catalyseur 1 à 5 % en poids de chlore par rapport au poids de mordénite, le composé chloré etant choisi dans le groupe constitué par l'acide chlorhydrique, le tétrachlorure de carbone, le dichloropropane, le chloroforme et le dichloroéthane.

13.- Procédé selon l'une des revendications 1 à 12 pour l'isomérisation d'une coupe d'hydrocarbures riche en n-paraffines ayant de 4 à 7 atomes de carbone par molécule, en présence d'un catalyseur renfermant au moins un métal du groupe VIII de la classification périodique des éléments supporté par au moins une mordénite acide, ayant une teneur en sodium inférieure à 0,2 % en poids par rapport au poids de la mordénite sèche, adsorbant des molécules de diamètre cinétique supérieur à environ $6,6 \times 10^{-10}$ m, ayant un volume de maille V de la maille élémentaire compris entre 2,73 et 2,78 $nm^3$, une capacité d'adsorption de benzène supérieure à 5 % poids par rapport au poids de mordénite sèche, le procédé étant caractérisé en ce que :

(a) on met en contact ledit catalyseur avec un gaz contenant au moins de l'oxygène moléculaire et 0,01 à 5 % en poids d'eau à une température que l'on élève progressivement jusqu'à ce qu'elle se trouve comprise entre 200 et 550°C, puis on poursuit l'addition dudit gaz, à une température comprise entre 200 et 500°C, en y ajoutant du chlore ou un composé chloré de façon à introduire au total, dans le catalyseur, 0,5 à 10 % en poids de chlore par rapport au poids de la mordénite contenue dans le catalyseur,

(b) on réduit le catalyseur obtenu à l'étape précédente à l'aide d'un gaz contenant au moins un composé réducteur, à une température comprise entre 300 et 750°C pendant un temps suffisant pour que la concentration en composé réducteur soit sensiblement la même à l'entrée et à la sortie de la zone où s'effectue la réduction du catalyseur,

(c) on soumet à une réaction d'isomérisation, en présence du catalyseur réduit à l'étape précédente, ladite coupe d'hydrocarbures, en présence d'hydrogène, à une température comprise entre 200 et 350°C environ, sous une pression partielle d'hydrogène comprise entre 0,1 et 7 MPa, avec une vitesse spatiale horaire de la charge d'hydrocarbures comprise entre 0,1 et 20 $h^{-1}$ et un rapport molaire $H_2$/charge d'hydrocarbures compris entre 0,2 et 20,

(d) à l'issue de la réaction d'isomérisation, on soumet une partie au moins du catalyseur à une combustion à l'aide d'un gaz contenant de l'oxygène à une température contrôlée inférieure à 550°C,

(e) on soumet le catalyseur issu de l'étape (d) à une oxychloration à une température d'environ 200 à 500°C à l'aide d'un mélange gazeux contenant de l'oxygène, de l'eau et au moins un agent choisi dans le groupe constitué par le chlore et les composés chlorés, la quantité de chlore ou de composé(s) chloré(s) employée représentant au total de 0,5 à 10 % en poids calculé en poids de chlore par rapport au poids de mordénite sèche et

(f) on traite à l'hydrogène et on recycle une partie au moins du catalyseur oxychloré vers l'étape (c).

14.- Procédé selon la revendication 13 dans lequel, à l'étape (a), on chauffe jusqu'à une température comprise entre 350 et 400°C et dans lequel ensuite on ajoute du chlore ou un composé chloré de façon à introduire dans le catalyseur 1 à 5 % en poids de chlore par rapport au poids de mordénite, le composé chloré etant choisi dans le groupe constitué par l'acide chlorhydrique, le tétrachlorure de carbone, le dichloropropane, le chloroforme et le dichloroéthane, et procédé dans lequel à l'étape (d), la combustion est conduite de manière à ce que la température reste inférieure à environ 500°C et à ce que la teneur pondérale du coke résiduel sur le catalyseur après la combustion, soit inférieure à 20 % de la teneur pondérale en coke du catalyseur avant combustion.

15.- Procédé selon la revendication 14 dans lequel le gaz employé pour la combustion du coke contient de 0,1 à 30 % en poids d'oxygène et dans lequel dans le mélange gazeux, employé dans l'étape (e) d'oxychloration, la quantité de chlore ou de composé chloré employée représente au total de 0,1 à 5 % en poids calculé en poids de chlore par rapport au poids de mordénite sèche.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 473 657  (H.F. TSE) <br> * Revendications 1,3,5-7 * <br> --- | | B 01 J   29/38 <br> C 07 C    5/27 <br> C 10 G   45/64 |
| A | BE-A-  664 201  (ESSO) <br> * Pages 7-15 * <br> --- | | |
| A | FR-A-2 512 356  (ELF) <br> --- | | |
| A | EP-A-0 142 352  (EXXON) <br> * Revendications 1,3,5,7-12,15-27 * <br> --- | | |
| A | FR-A-2 325 289  (IFP) <br> * Revendication 1 * <br> --- | | |
| P,A | US-A-4 657 874  (W. BORGHARD) <br> * Colonne 2, ligne 59 - colonne 4, ligne 42 * <br> --- | | |
| P,A | US-A-4 645 751  (S.B. McCULLEN) <br> * Colonne 3, ligne 22 - colonne 5, ligne 54 * <br> ----- | | |

DOMAINES TECHNIQUES
RECHERCHES (Int. Cl.4)

B 01 J   29/00

Le présent rapport a été établi pour toutes les revendications

| Lieu de la recherche | Date d'achèvement de la recherche | Examinateur |
|---|---|---|
| LA HAYE | 07-06-1988 | DEVISME F.R. |